# EUROPEAN PATENT SPECIFICATION

(11) **EP 1 507 838 B1**
(45) Date of publication and mention of the grant of the patent: **14.09.2011**
(21) Application number: 02780661.1
(22) Date of filing: 15.11.2002
(51) Int. Cl.: C10G 1/00

(54) **A PROCESS TO PRODUCE A DILUTE ETHYLENE STREAM AND A DILUTE PROPYLENE STREAM**
VERFAHREN ZUR HERSTELLUNG EINES VERDÜNNTEN ETHYLENSTROMS UND EINES VERDÜNNTEN PROPYLENSTROMS
PROCEDE DE PRODUCTION D'UN COURANT D'ETHYLENE DILUE ET D'UN COURANT DE PROPYLENE DILUE

(30) Priority: 16.11.2001 US 992445; 27.02.2002 US 83934
(43) Date of publication of application: 23.02.2005
(60) Divisional application: 10178012.0
(73) Proprietor: CHEVRON PHILLIPS CHEMICAL COMPANY LP, The Woodlands, TX 77380 (US)
(72) Inventor: PORTER, Rodney, L., Pearland, TX 77584 (US); BALINSKY, Anne, M., Kingwood, TX 77345 (US); WEBER, Eric, P., Kingwood, TX 77345 (US)
(74) Representative: Nash, David Allan
(86) International application number: PCT/US2002/036535
(87) International publication number: WO 2003/044125

(56) References cited:
- EP-A- 0 825 245
- WO-A-97/33953
- US-A- 4 121 917
- US-A- 5 090 977
- US-A1- 2001 044 565
- US-A1- 2002 004 622

## Description

### FIELD OF THE INVENTION

This invention is related to the field of processes wherein a cracked gas stream is separated to produce dilute olefin streams to bused as feedstocks to produce olefin-based derivatives. Specifically, this invention is related to the field of processes wherein a cracked gas stream is separated to produce a dilute ethylene stream and a dilute propylene stream to be used as feedstocks for producing olefin-based derivative. More specifically; the dilute ethylene stream is used as a feedstock to produce ethylbenzene, and the dilute propylene stream is used as a feedsfock to produce cumene, acrylic acid and propylene oxide or other propylene based derivatives

### BACKGROUND OF THE INVENTION

Feedstock costs in the chemical industry comprises a significant portion of the manufacturing costs. Continuous research is being conducted to lower these costs by utilizing lower cost feed sources. The alkylation of benzene and other aromatics is one area where dilute olefin streams are employed to reduce feed related manufacturing costs. For example in the production of ethylbenezene, a raw material for the production ot styrene, the off gas from a fluidize catalytic cracking unit (FCC) can be successfully employed as a cost advantaged ethylene source. The: FCC quantities of diluents in the FCC off-gas, such as, for example, hydrogen and methane, the alkylation section of the ethylbenzene unit requires that some of the equipment be oversized. Additionally, the hydrogen sulfide content of the FCC off-gas necessitates its removal in a gas pretreatment section and subsequent compression before it can be routed to the alkylation reactor. The requirements, of having oversized equipment and gas pre-treatment followed by compression greatly increase the capital costs associated with an ethylbenzene unit utilizing FCC off-gas as its feedstock compared to a conventional ethylbenzene unit that utilizes high purity, polymer grade ethylene.

There is a need in the chemical industry to reduce feedstock costs by utilizing dilute olefin streams at olefins-based derivative units rather than polymer grade olefin feedstocks. To fulfill this need, the inventors provide this inventive process. This process reduces the amount of equipment traditionally required for the production of ethylene. An example of some of the equipment that has been eliminated is the ethylene refrigeration compressor, demethanizer, cold box system, and C₂ and C₃ splitters. Additionally, some equipment is smaller than with conventional crackers of comparable scale. The propylene refrigeration system is reduced in size over that of a conventional cracker. This invention also benefits the olefin-based derivative units that produce, for example, ethylbenzene, cumene, acrylic acid, and propylene oxide. One of the benefits is the pretreatment normally required for the olefins-based derivative units is not necessary in this inventive process because treatment has already been accomplished in the process to produce the dilute olefins stream. In others words, this inventive process to produce dilute olefin streams and route these stream to olefins-based derivative units has a reduced capital cost over a traditional FCC off-gas process since all pretreatment and compression is handled by the dilute olefins process.

### SUMMARY OF THE INVENTION

An object of this invention is to provide a process to produce a dilute ethylene stream and a dilute propylene stream from a cracked gas stream.

Another object of this invention is to provide a process to produce the dilute ethylene stream and the dilute propylene stream from a cracked gas stream generated by the steam cracking of C₂ and higher hydrocarbons.

Another object of this invention is to provide a process to produce the dilute ethylene stream and dilute propylene stream wherein these streams are utilized to produce olefin-based derivatives.

Another object of this invention is to provide a process to produce a dilute ethylene stream wherein the dilute ethylene stream is used as a feedstock to produce ethylbenzene.

Another object of this invention: is to provide a process to produce a dilute ethylene stream wherein the ethylbenzene unit utilizing the dilute ethylene stream does not contain pretreatment and compression zones.

Another object of this invention is to provide a process to produce a dilute propylene stream wherein the dilute propylene stream is used as a feedstock to produce cumene, acrylic acid, propylene oxide and other propylene derivatives.

Yet another object of this invention is to produce cumene, acrylic acid, and propylene oxide and other propylene derivatives without a pretreatment unit.

Thus, in accordance with a first aspect, there is provided a process for producing a dilute ethylene stream from a cracked gas stream comprising hydrogen, methane, L₂ hydrocarbons, L₃ hydrocarbons and heavier constituents, said process comprising the following steps in the order named
(1) separating said cracked gas stream comprising hydrogen, methane, L₂ hydrocarbons, L₃ hydrocarbons and heavier constituents in a deethanizer zone to produce a C₂-stream and a C₃+ stream;
(2) hydrogenating said C₂₋ stream in a hydrogenation zone to remove a portion of the acetylene to produce said dilute ethylene stream; and
(3) routing said C₃+ stream to storage or other process unit.

In an embodiment of the first aspect, said C₂- stream is compressed in a C₂- stream-compression zone to form a pressurized C₂- stream which is hydrogenated in step (2).

In accordance with a second aspect, there is provided process for producing a dilute ethylene stream from a cracked gas stream comprising hydrogen, methane, C₂ hydrocarbons, C₃ hydrocarbons and heavier constituents, said process comprising the following steps in the order named:
(1) hydrogenating a portion of the acetylene in said cracked gas stream in a hydrogenation zone to produce reduced acetylene cracked gas stream;
(2) separating said reduced acetylene cracked gas stream in a deethanizer zone to produce said dilute ethylene stream and a C₃+ stream;
(3) routing said C₃+ stream to storage or other process unit.

The process according to the first or second aspect may be additionally for producing a dilute propylene stream from the cracked gas stream, wherein in step (3) said C₃+ stream is separated in a depropanizer zone to produced a C₃ stream and a C₄+ stream; and wherein said C₃ stream is reacted in a MAPD reactor zone to convert a portion of methylacetylene and propadiene to propylene and propane to produce said dilute propylene stream.

In accordance with a third aspect, there is provided process for producing a dilute ethylene, stream and a dilute propylene stream, said process comprising the following steps in the order named:
(1) separating a cracked gas stream comprising hydrogen, methane, C₂ hydrocarbons, C₃ hydrocarbons and heavier constituents, in a depropanizer zone to form a C₃- stream and a C₄+stream;
(2) separating said C₃- stream in a deethanizer zone to form a C₂- stream and a C₃ steam;
(3) hydrogenating a portion of the acetylene in said C₂- stream in a hydrogenation zone to produce a dilute ethylene stream; and
(4) reacting said C₃ stream in a MAPD zone to convert a portion of methylacetylene and propadiene to propylene and propane to produce said dilute propylene stream.

In an embodiment of the first, second or third aspects, said cracked gas stream is produced by a process comprising:
(1) heating a hydrocarbon feed in a cracking zone to form a raw cracked gas stream; wherein said raw cracked gas stream comprises hydrogen, methane, C₂ hydrocarbons, C₃ hydrocarbons and heavier constituents;
(2) quenching said raw cracked gas stream in a quenching zone to produce a quenched, cracked gas stream;
(3) compressing said quenched, cracked gas stream in a cracked gas stream-compression zone to form a pressurized, cracked gas stream;
(4) deacidifying said pressurized, cracked gas stream in a deacidifying zone to remove a portion of the hydrogen sulfide to form a wet cracked gas stream; and
(5) drying said wet cracked gas stream in a drying zone to form a cracked gas stream.

In embodiment in which a C₄+ stream is produced, the process may, further comprise separating said C₄+ stream in a debutanizer zone to produce a C₄ streani and a C₃+ stream.

The process of the first, second or third aspects may further comprise passing said dilute ethylene stream to a dilute ethylene derivative unit.

In an embodiment, said dilute ethylene derivative unit produces ethylbenzene.

In embodiment in with a dilute propylene stream is produced, the process may, further comprise passing said dilute propylene stream to a dilute propylene derivative unit.

In an embodiment, said dilute propylene derivative unit produces cumene, acrylic, acid or propylene oxide.

In embodiments in which a C₅+ stream is produced, the process further comprises treating said C₅+ stream in a. hydrotreating zone to produce a C₅ diolefins stream, a BTX stream, a DCPD stream and a fuel oil stream.

In embodiments in which said cracked gas stream is produced by heating a hydrocarbon feed in a cracking zone, said hydrocarbon feed may be selected from the group consisting of ethane, propane, butanes, pentanes, naphtha, and mixtures thereof. In

another embodiment, said hydrocarbon feed consists essentially of C₅ hydrocarbons.

In an embodiment of the first, second or third aspects, a propylene oxide stream is produced by a process comprising the following steps:
(1) reacting said dilute ethylene with benzene in an ethylbenzene reactor zone to form an ethylbenzene stream;
(2) oxidizing said ethylbenzene stream with air in an EB oxidation zone to form a EBHP stream;
(3) reacting said EBHP stream with a dilute: propylene stream in a propylene epoxidation zone to form an impure propylene oxide stream;
(4) separating said impure propylene oxide streams in a produce separator zone to form a raw propylene oxide stream, a MBN/ACP stream, a tail gas stream, and a residue steam; and
(5) separating said raw propylene oxide stream in a propylene: oxide separations zone to form an impurities stream and said propylene oxide stream; and
(6) reacting said MBN/ACP stream in a styrene production and separation zone to form a styrene stream, a fuel stream, and a wastewater stream.

In embodiment of the first or second aspects described above in which a C₃ stream and a C₄+ stream is produced, or in an embodiment of the third aspect, an acrylic acid stream is produced by a process comprising the following steps:
(1) oxidizing said dilute propylene stream in a oxidation reactor zone to form a aqueous acrylic acid stream and a vent gas stream; and
(2) seperating said aqueous acrylic acid stream in a recovery and purification zone to form said acrylic acid stream and a mixed acid/ester waste stream.

In embodiment of the first or second aspects described above in which a C₃ stream and a C₄+ stream is produced, or in an embodiment of the third aspect, a cumene stream is produced by a process comprising the following steps:
(1) reacting a dilute propylene stream and a benzene feed stream in a dilute propylene alkylation zone to produce a raw cumene stream;
(2) separating said raw cumene stream in a cumene separations zone to form a. benzene stream a heavies steam said cumene stream, a dipropyl benzene stream and a propane stream.
(3) transalkylating said benzene stream an dipropyl benzene stream in a transalkylation zone to form a transalkylated cumene rich stream;
(4) separating said transalkylated cumene-rich stream in said cumene separations zone to produce said cumene stream, said propane stream, said heavies stream and said benzene stream; and
(5) optionally, recycling a portion of said benzene stream to said dilute propylene alkylation zone

In embodiment of the first or second aspects described above in which a C₃ stream and a C₄+ stream is produced, or in an embodiment of the third aspect, a ethylbenzene stream is produced by a process comprising the following steps;
(1) reacting a dilute ethylene stream and a benzene stream in an alkylation reactor zone to form an ethylbenzene rich stream
(2) separating said ethylbenzene rich stream in a ethylbenzene separation zone to form a separations benzene recycle stream, a separations tail gas stream, a diethylbenzene and polyethylbenzene stream, and a ethylbenzene stream;
(3) reacting said separations benzene recycle stream in an ethylbenzene transalkylation reactor zone to produce said ethylbenzene rich stream; and
(4) optionally, recycling a portion of said separations benzene recycle stream to said dilute propylene alkylation reactor zone.

These object, and other objects, will become more apparent to others with ordinary skill in the art after reading this disclosure.

### Brief Description of Drawings

- FIG 1.: A diagram showing an embodiment of the process to produce dilute propylene and dilute ethylene.
- FIG 2.: A diagram showing the preferred method of producing cracked gas feed.
- FIG 3.: A. diagram showing another embodiment of the process to produce dilute 15 propylene and dilute ethylene with a second compression zone.
- FIG 4.: A diagram showing another embodiment of the process to produce dilute ethylene and dilute propylene with a hydrogenation zone before the deethanizer zone.
- FIG 5.: A diagram for a process to produce dilute ethylene.
- FIG 6.: A diagram for a process to produce dilute ethylene with a second compression zone.
- FIG 7.: A diagram for a process to produce dilute ethylene with a hydrogenation zone before the deethanizer zone.
- FIG 8.: A diagram for a process to produce dilute ethylene and dilute propylene with a depropanizer zone as the first separation.
- FIG 9.: A diagram of a process to produce propylene oxide.
- FIGS 10.: A diagram of a process to produce acrylic acid.
- FIG 11.: A diagram of a process to produce cumene.
- FIG 12.: A diagram of a process to produce ethylbenzene.

### DETAILED DESCRIPTION OF THE INVENTION

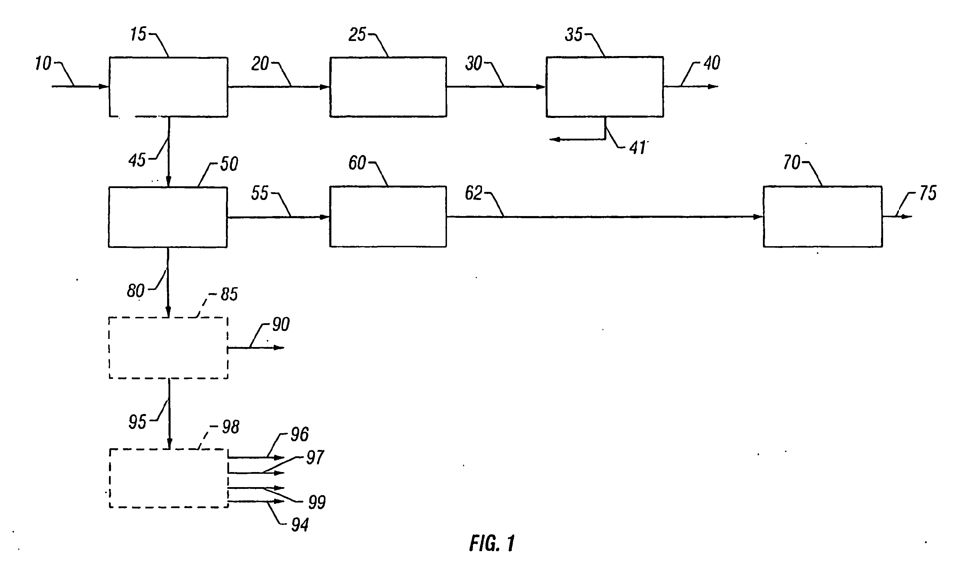

In a first embodiment of this invention, a process for producing a dilute ethylene stream and a dilute propylène stream from a cracked gas stream is provided as shown in Figure 1.

Step (1) is separating the cracked gas stream in line 10 in a deethanizer zone 15 to produce a C₂- stream in line 20 and a C₃+ stream in line 45. The deethanizer zone 15 comprises a fractionator sufficient to produce the C₂- stream in line 20 and a C₃+ stream in line 45. The C₂- stream comprises hydrogen, methane, ethane, acetylene and ethylene. The C₃+ stream comprises C₃ hydrocarbons and heavier constituents. The cracked gas stream in line 10 comprises hydrogen, methane, C₂ hydrocarbons, C₃ hydrocarbons, and heavier constituents, and can be produced by any means known in the art.

Step (2) is hydrogenating the C₂- stream in line 20 in a hydrogenation zone 25 to remove a portion of the acetylene to produce the dilute ethylene, stream in line 30. Hydrogenation in the hydrogenating zone 25 can be completed by any means know in the art. For example, an acetylene reactor containing catalyst can be utilized to hydrogenate a portion of the acetylene Typically, Group VIII metal hydrogenation catalysts are utilized. Hydrogenation catalysts are disclosed in U.S. Patent. Numbers 3,679,762; 4,571,442; 4,347,392; 4,128,595; 5,059,732; 5,488,024; 5,489,565; 5,520,550; 5,583,274; 5,698,752; 5,585,318; 5,587,348; 6,127,310 and , 702,956. Generally, the amount of acetylene remaining in the dilute ethylene stream in line 30 is in a range of less than about 5 ppm by weight, preferably, in a range of 0.5 ppm to 3 ppm by weight.

The temperature and pressure in the hydrogenation zone 25. is that which is sufficient to substantially hydrogenate the acetylene in the C₂- stream in line 20. Preferably, the hydrogenating occurs at a temperature in a range of about 50° F (10°C) to about 400°F (204°C) and at pressure in a range of about 350 psi (3.4 MPa) to about 600 psi (4.1 MPa1) an absolute

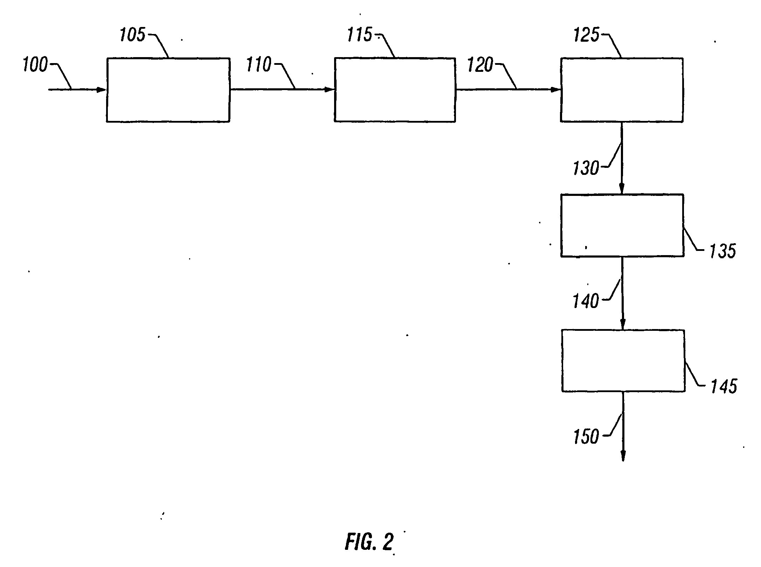

Generally, the amount of ethylene in the dilute ethylene stream in line 30 is in a range of about 30% to about 60% by weight, preferably, 40% to 60% by weight. The dilute ethylene stream in line 30 then can be routed to a dilute ethylene derivative unit 35 to produce different chemicals in line 40 including, but not limited to, ethylbenzene. Preferably, the dilute ethylene stream in line 30 is routed to an ethylbenzene unit. The ethylbenzene unit can utilize any process known in the art. For example, a Friedel-Crafts alkylation reaction of benzene by ethylene is used. Optionally, an effluent gas stream in line 41 from the dilute ethylene derivative unit 3:5 can be recycled to a cracking zone 105, shown in Figure 2, to produce more dilute ethylene. The composition of the effluent gas stream can vary widely depending on the predominant hydrocarbons feed initially fed to the cracking zone 105. Typically, the effluent gas stream comprises hydrogen, methane, and other light hydrocarbons. Hydrogen and methane may need to be removed from the dilute process stream prior to recycle. This removal can be accomplished by separation membranes, separators, or other equipment.

Step (3) is separating the C₃+ stream in line 45 in a depropanizer zone 50 to produce a C₃ stream ion line 55 and a C₄+ stream in line 80. The depropanizer zone 50 comprises a fractionator sufficient to produce the C₃ stream in line 55 and a C₄+ stream in line 80. The C₃ stream in line 55 comprises propane, propylene, methylacetylene and propadiene. The amount of propylene in the C₃ stream in line 55 is in a range of about 55% to about 98% by weight, preferably, in a range of 85% to 96% by weight. The: C₄+ stream in line 80 comprises C₄ hydrocarbons and heavier hydrocarbon constituents.

Step (4) is reacting the G3 stream in line 55 in a MAPD reactor zone 60 to remove a portion of methylacetylene and propadiene to produce the dilute propylene stream in line 62. The hydrogenation process for the reduction of MAPD occurs in the MAPD reactor zone 60. can be completed by any means known in the art. Generally, the amount of methylacetylene and propadiene remaining in the dilute propylene stream in line 62 is less than 2 ppm by weight

The dilute propylene stream in line 62 can be routed to an dilute propylene derivative unit 70 to produce different dilute propylene darivatives. For example, the dilute propylene stream in line 62 can be routed to a process to produce cumene, propylene oxide or acrylic acid in line 75. Cumene can be produced by any process known in the art. For example, a Friedel-Crafts alkylation reaction of benzene by propylene is used to produce cumene. Cumene then can be used to produce other products, such as, for example, phenols.

Optionally, the C₄+ stream in line 80 is separated in a debutanizer zone 85 to produce a. C₄ stream in line 90 and a C₅+ stream in line 95. The debutanizer zone 85 comprises a fractionator sufficient to produce the C₄ stream in line 90 and a C₅+ stream in line 95. The C₄ stream in line 90 comprises C₄ hydrocarbons. The C₅+ stream in line 95 comprises C₅ hydrocarbons and heavier hydrocarbon constituent.

Optionally, the C₅+ stream in line 95 is treated in a hydrotreating zone 98 to produce a C₅ diolefins stream in line 96, a benzene-toluene-xylenes (BTX) stream in line 99, a dicyclopentadiene (DCPD) stream in line 97 and a fuel oil stream in line 94. The treatment of the C₅+ stream in the hydrotreating zone 98 can be accomplished by any means known in the art For example, U.S patent number 6,258,989 discloses a hydrotreating zone that can be utilized in this invention. The C₅ diolefins stream in line 96 comprises C₅ hydrocarbons, and the BTX stream in line 99 comprises benzene, toluene, and xylenes. The DCPD stream in line 97 comprises dicyclopentadiene, and the fuel oil stream in line 94 Comprises C₈+ hydrocarbons.

In a second embodiment of the invention, the cracked gas stream utilized as the feedstock in this process can be produced by any process known in the art. A preferred process for producing the cracked gas stream is provided as shown in Figure 2.

Step (1) is heating a hydrocarbon feed in line 100 in a cracking zone 105 to produce a raw cracked gas stream in line 110. Generally, the hydrocarbon feed in line 100 comprises at least one hydrocarbon selected from the group consisting of ethane, propane, butane, pentana; naphtha, gas condensates, gas oils, and mixtures thereof. Preferably, a majority of the hydrocarbon feed in line 100 consists of C₅ hydrocarbons and higher hydrocarbons.

The cracking zone 105 comprises at least one radiant furnace reactor capable of producing the raw cracked gas stream in line 110. Typically, dilution stream is added to the radiant furnace reactors to reduce coking and to reduce the partial pressure of the hydrocarbon feed, thus increasing ethylene yield. Radiant furnace reactors are disclosed in U.S. Patent Numbers 5,151.158; 4,780,196; 4,499,055; 3,274,978; 3,407,789; and 3,820,955 .

The raw cracked gas stream in line 1 to comprises hydrogen, methane, C₂, hydrocarbons, C₃ hydrocarbons, and heavier constituents. Generally, the raw cracked gas stream in line 110 comprises at least about 10% by weight ethylene, preferably, at least about 20% by weight ethylene, and most preferably, at least about 30% by weight ethylene. For example, the raw cracked gas stream in line 110 comprises about 1 to about 5 weight percent hydrogen, about 3 to about 25 weight percent methane, less than about 1 weight percent acetylene, about 25 to about 35 weight percent ethylene, about 3 to about 45 weight percent ethane, and up to about 55 weight percent:C₃+ hydrocarbons, depending on the hydrocarbon feed.

Step (2) is quenching the raw cracked gas stream in line 1 iso in.a quenching zone 115 to produce a quenched, cracked gas stream in line 120. Typically, the raw cracked gas stream in line 110 is quenched in quenching, zone 115 to a temperature, below which the cracking reaction substantially stops in order to present coking. Generally, the raw cracked gas stream in line 110 is cooled to a temperature below about: 1100°f (593°C) to substantially stop the cracking reaction. Preferably, the raw cracked gas stream in line 110 is cooled to a temperature in a range of about 85 to about 225°F to form the quenched cracked gas stream in line 120. Quenching can be effected by any means knows in the art. For example, the raw cracked gas stream in line.110 can be passed to a. quench boiler and quench tower where fuel oil and dilution stream can be removed. Method for cooling a raw cracked gas stream are disclosed in U.S. Patents 3,407,798; 5,427,655; 3,392,211; 4,3351,275; and 3,403,722.

Step (3) is compressing the quenched, cracked gas stream in line 120 in a first compression zone 125 to produce a pressurized, cracked gas stream in line 130. The gange pressure of the pressurized, cracked gas stream in line 130 is in a range of about 150 psi (1.0 MPa) to about 650 psi (4.5MPa). The first compression zone 125 comprises at least one gas compressor. Any gas compressor known in the art can be utilized.

Step (4) is deacidifying the pressurized, cracked gas stream in line 130 in a deacidifying zone 135 to remove a portion of the hydrogen sulfide and carbon dioxide to form a wet cracked gas stream in line 140. Generally, the wet cracked gas stream in line 140 has a hydrogen sulfide concentration less than about 0.1 ppm by weight, preferably, in a range of 25 to 100 ppb by weight. Generally, the wet cracked gas stream has a carbon dioxide concentration of less than about 5 ppm by weight. The hydrogen sulfide can be removed in the deacidifying zone 135 by any means known in the art. For example, diethanolamine or caustic contactors can be used to remove hydrogen sulfide and carbon dioxide.

Step (5) is drying the wet cracked gas stream in line 140 in a drying zone 145 to produce the cracked gas stream in line 150. Generally, the water content of the clacked gas stream in line 150 is sufficiently dry to prevent downstream operational problems. Preferably, the water content of the cracked gas stream in line 150 is less than 10 ppm by weight. Drying in drying zone 145 can be accomplished by any means known in the art. For example, molecular sieve beds can be utilized to remove water from the wet cracked gas stream in line 140.

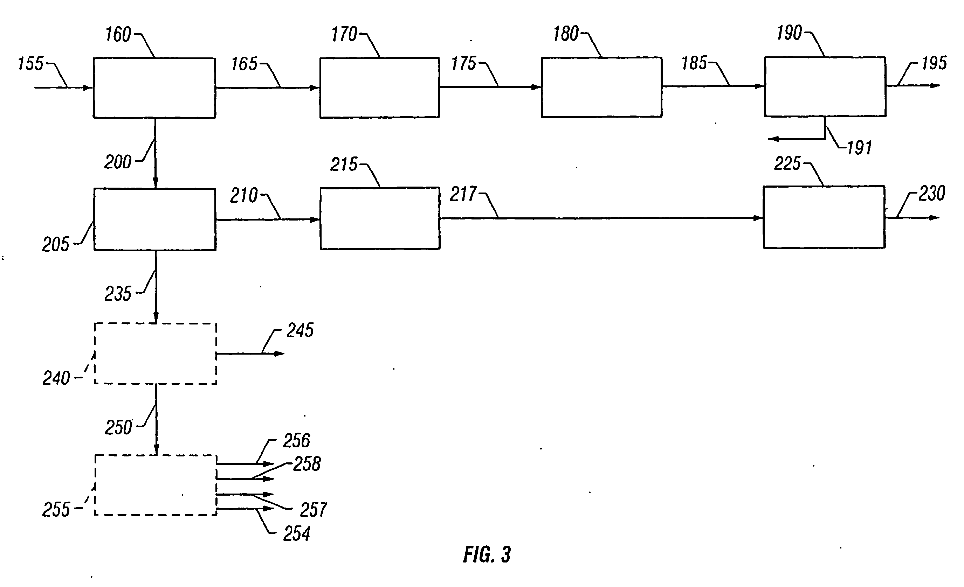

In a third embodiment of this invention, a process for producing a dilute ethylene stream and dilute propylene stream from a cracked gas stream is provided as shown in Figure 3.

Step (1) is separating the cracked gas stream in line 155 in a deethanizer zone 160 to produce a C₂- stream in line 165 and a C₃+ stream in line 200. The deethanizer zone 160 comprises a fractionator sufficient to produce the C₂- stream in !me 165 and a C₃+ stream in line 200. The C₂- stream comprises hydrogen, methane, ethane, acetylene and ethylene. The C₃+ stream comprises C₃ hydrocarbons and heavier constituents.

Step (2) is compressing the C₂- stream in line 165 in a second compression zone 170 to produce a pressurized, C₂- stream in line 175. The gange pressure of the pressurized, C₂- stream in line 175 is in a range of about 150 to about 650 psi preferably, in a range of 200 to 650 psi (1.4-4.5MPa). The second compression zone 170 comprises a gas compressor and related equipment. Any gas compressor known in the art can be utilized.

Step (3) is hydrogenating the pressurized C₂- stream in line 175 in a hydrogenation zone 180. to remove a portion of the acetylene to produce the dilute ethylene stream in line 185. The hydrogenation, zone 180 is the same as previously described in the first embodiment.

Generally, the amount of ethylene in the dilute ethylene stream in line 185 is in a range of about 30% to about 60% by weight, preferably, 40% to 60 % by weight. The dilute ethylene stream in line 185 then can be routed to an dilute ethylene derivative unit 190 to produce different chemicals in line 195 including, but not limited to, ethylbenzene. The dilute ethylene derivative unit 190 is the same as dilute ethylene derivative unit 35 previously described in the first embodiment. Optionally, an effluent gas stream in line 191 from the dilute ethylene derivative unit 190 can be recycled to a cracking zone 105 in Figure 2.

Step (4) is separating the C₃+ stream in line 200 in a depropanizer zone 205 to produce a C₃ stream in line 210 and a C₄+ stream in line 235. The depropanizer zone 205 and the C₃ stream and the C₄+ stream are the same as previously described in the first embodiment.

Step (5) is reacting the C₃ stream in line 210 in a MAPD reactor zone 215 to remove a portion of methylacetylene and propadiene to produce the dilute propylene stream in line 217. The MAPD reactor zone 215 is the same as MAPD reactor zone 60 previously described in the first embodiment. The dilute propylene stream is the same as previously described in the first embodiment.

The dilute propylene stream in line 217 then can be routed to a dilute propylene derivative unit 225 to produce different dilute propylene derivatives in line 230. The dilute propylene derivative unit 225 is the same as dilute propylene derivative unit 70 previously described in the first embodiment.

Optionally, the C₄+ stream in line 235 is separated in a debutanizer zone 240 to produce a C₄ stream in line 245 and a C₅+ stream in line 250. The debutanizer zone 240 comprises a fractionator sufficient to produce the C₄ stream in line 245 and a C₅+ stream in line 250. The debutanizer zone 240 and the C₄ stream in line 245 and the C₅+ stream in line 250 are the same as previously described in the first embodiment.

Optionally, the C₅+ stream in line 250 is treated in a hydrotreating zone 255 to produce a C₅ diolefins stream in line 256, a BTX stream in line 257, a DCPD stream in line 258, and a fuel oil stream in line 254. The hydrotreating zone 255, the C₅ diolefins stream in line 256, the BTX stream in line 257, and the DCPD stream in line 258 and the fuel oil stream in line 254 are the same as previously described in the first embodiment.

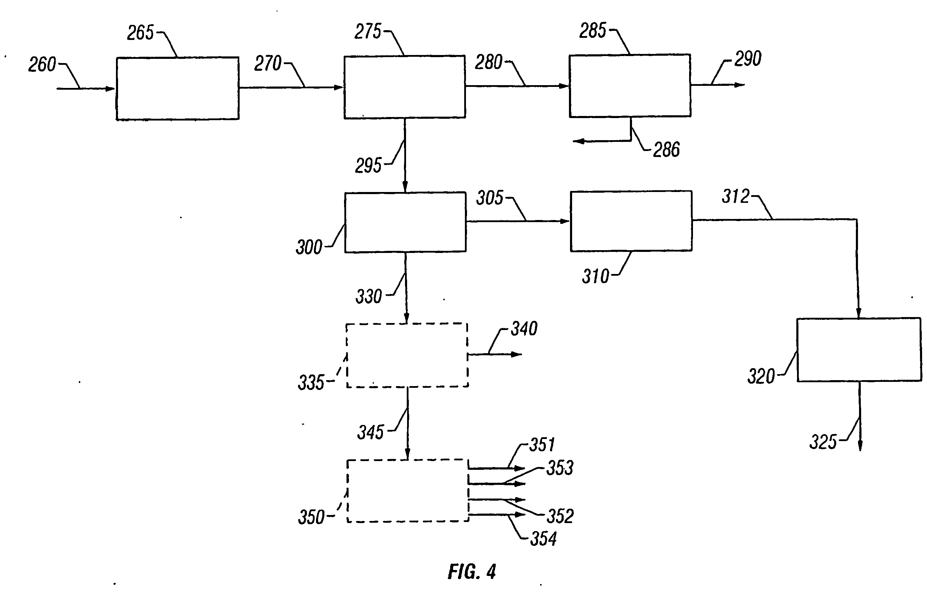

In a fourth embodiment of this invention, a process for producing a dilute ethylene and dilute propylene stream from a cracked gas stream is provided as shown in Figure 4.

Step (1) is hydrogenating the cracked gas stream in line 260 in a hydrogenation zone 265 to remove a portion of the acetylene to produce a reduced acetylene cracked gas stream in line 270. The hydrogenation zone 265 is the same as previously described in the first embodiment.

Step (2) is separating the reduced acetylene cracked gas stream in line 270 in a deethanizer zone 275 to produce the dilute ethylene stream in line 280 and a C₃+ stream in line 295. The deethanizer zone 275 comprises a fractionator sufficient to produce the dilute ethylene stream in line 280 and a C₃+ stream in line 295. The deethanizer zone 275, dilute ethylene stream in line 280 and C₃+ stream in line 295 are the same as previously described in the first and third embodiments.

Generally, the amount of ethylene in the dilute ethylene stream in line 280 is in a range of about 30% to about 60% by weight, preferably, 40% to 60 % by weight. The dilute ethylene stream in line 280 then can be routed to an dilute ethylene derivative unit 285 to produce different chemicals in line 290 including, but not limited to, ethylbenzene. The dilute ethylene derivative unit 285 is the same as dilute ethylene derivative unit 35 previously described in the first embodiment. Optionally, an effluent gas stream in line 286 from the dilute ethylene derivative unit 285 can be recycled to a cracking zone 105 in Figure 2.

Step (3) is separating the C₃+ stream in line 295 in a depropanizer zone 300 to produce a C₃ stream in line 305 and a C₄+ stream in line 330. The depropanizer zone 300, the C₃ stream in line 305, and the C₄+ stream in line 330 are the same as previously described in the first embodiment.

Step (4) is reacting the C₃ stream in line 305 in a MAPD reactor zone 310 to remove a portion of methylacetylene and propadiene to produce the dilute propylene stream in line 312. The MAPD reactor zone 310 and the dilute propylene stream in line 312 is the same as previously described in the first embodiment.

The dilute propylene stream in line 312 can be routed to a dilute propylene derivative unit 320 to produce different dilute propylene derivatives line 325. The dilute propylene derivative unit 320 is the same as previously described in the first and third embodiments.

Optionally, the C₄+ stream in line 330 is separated in a debutanizer zone 335 to produce a C₄ stream in line 340 and a C₅+ stream in line 345. The debutanizer zone 335 comprises a fractionator sufficient to produce the C₄ stream in line 340 and a C₅+ stream in line 345. The debutanizer zone 335, the C₄ stream in line 340, and the C₅+ stream in line 345 are the same as previously described in the first and third embodiments.

Optionally, the C₅+ stream in line 345 is treated in a hydrotreating zone 350 to produce a C₅ diolefins stream in line 351, a BTX stream in line 352, a DCPD stream in line 353, and a fuel oil stream in line 354. The hydrotreating zone 350, the C₅ diolefins stream in line 351, the BTX stream in line 352, the DCPD stream in line 353, and the fuel oil stream in line 354 are the same as previously described in the first embodiment.

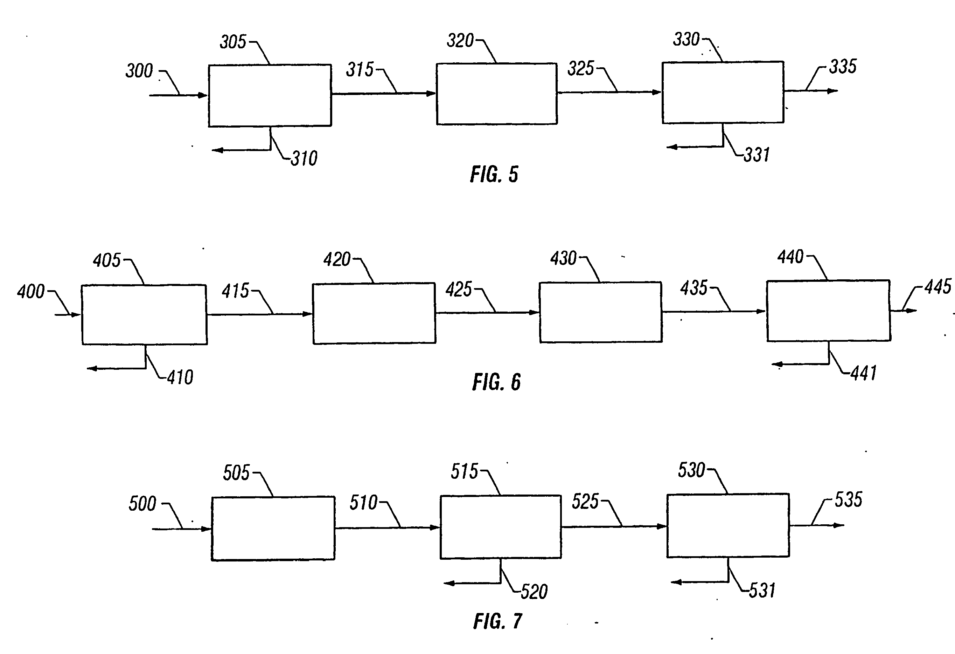

In a fifth embodiment of this invention, a process for producing a dilute ethylene stream from a cracked gas stream is provided as shown in Figure 5.

Step (1) is separating the cracked gas stream in line 300 in a deethanizer zone 305 to produce a C₂- stream in line 315 and a C₃+ stream in line 310. The deethanizer zone 305 comprises a fractionator sufficient to produce the C₂- stream in line 315 and a C₃+ stream in line 310. The C₂- stream comprises hydrogen, methane, ethane, acetylene and ethylene. The C₃+ stream comprises C₃ hydrocarbons and heavier constituents.

Step (2) is hydrogenating the C₂- stream in line 315 in a hydrogenation zone 320 to remove a portion of the acetylene to produce the dilute ethylene stream in line 325. The hydrogenation zone 320 and the dilute ethylene stream in line 325 are the same as previously described in the first embodiment.

Generally, the amount of ethylene in the dilute ethylene stream in line 325 is in a range of about 30% to about 60% by weight, preferably, 40% to 60 % by weight. The dilute ethylene stream in line 325 then can be routed to an dilute ethylene derivative unit 330 to produce different chemicals in line 335 including, but not limited to, ethylbenzene. The dilute ethylene derivative unit 330 is the same as dilute ethylene derivative unit 35 previously described in the first embodiment. Optionally, an effluent gas stream in line 331 from the dilute ethylene derivative unit 330 can be recycled to a cracking zone 105 in Figure 2.

Step (3) is routing the C₃+ stream in line 310 to storage or to other process units.

In a sixth embodiment of this invention, a process for producing a dilute ethylene stream from a cracked gas stream is provided as shown in Figure 6.

Step (1) is separating the cracked gas stream in line 400 in a deethanizer zone 405 to produce a C₂- stream in line 415 and a C₃+ stream in line 410. The deethanizer zone 405 comprises a fractionator sufficient to produce the C₂- stream in line 415 and a C₃+ stream in line 410. The C₂- stream comprises hydrogen, methane, ethane, acetylene and ethylene. The C₃+ stream comprises C₃ hydrocarbons and heavier constituents.

Step (2) is compressing the C₂- stream in line 415 in a second compression zone 420 to produce a pressurized, C₂- stream in line 425. The gange pressure of the pressurized, C₂- stream in line 425 is in a range of about 150 to about 650 psi(1·0-4·5MPa) preferably, in a range of200 to 650 psi(1·4-4·5 MPa). The second compression zone 420 comprises a gas compressor and related equipment Any gas compressor known in the art can be utilized.

Step (3) is hydrogenating the pressurized C₂- stream in line 425 in a hydrogenation zone 430 to remove a portion of the acetylene to produce the dilute ethylene stream in line 435. The hydrogenation zone 430 is the same as previously described in the first embodiment.

Generally, the amount of ethylene in the dilute ethylene stream in line 435 is in a range of about 30% to about 60% by weight, preferably, 40% to 60 % by weight. The dilute ethylene stream in line 435 then can be routed to an dilute ethylene derivative unit 440 to produce different chemicals in line 445 including, but not limited to, ethylbenzete. The dilute ethylene derivative unit 440 is the same as dilute ethylene derivative unit 35 previously described in the first embodiment. Optionally, an effluent gas stream in line 441 from the dilute ethylene derivative unit 440 can be recycled to a cracking zone 105 in Figure 2.

Step (4) is routing the C₃+ stream in line 410 to storage or to other process units.

In a seventh embodiment of this invention, a process for producing a dilute ethylene stream from a cracked gas stream is provided as shown in Figure 7.

Step (1) is hydrogenating the cracked gas stream in line 500 in a hydrogenation zone 505 to remove a portion of the acetylene to produce a reduced, acetylene cracked gas stream is line 510. The hydrogenation zone 505 is the same as previously described in the first and third embodiment.

Step (2) is separating the reduced acetylene cracked gas stream in line 510 in a deethanizer zone 515 to produce the dilute ethylene stream in line 525 and a C₃+ stream in line 520. The deethanizer zone 515 comprises a fractionator sufficient to produce the dilute ethylene stream in line 525 and a C₃+ stream in line 520. The deethanizer zone 515, dilute ethylene stream in line 525 and C3+ stream in line 520 are the same as previously described in the first embodiment.

Generally, the amount of ethylene in the dilute ethylene stream in line 525 is in a range of about 30% to about 60% by weight, preferably, 40%, to 60 % by weight The dilute ethylene stream in line 525 then can be routed to an dilute ethylene derivative unit 530 to produce different chemicals in line 535 including, but not limited to, ethylbenzene. Optionally, an effluent gas stream in line 531 can be recycled back to a cracking zone 105 shown in Figure 2 to produce more dilute ethylene. The dilute ethylene derivative unit 530: is the same as dilute ethylene derivative unit 35 previously described in the first embodiment.

Step (3) is routing the C₃+ stream in line 520 to storage or to other process units.

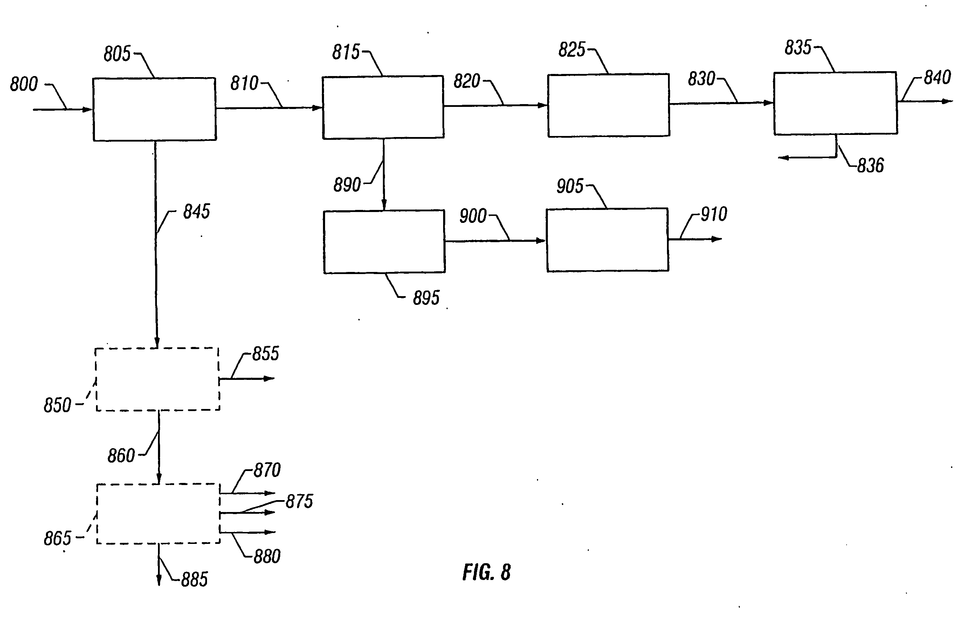

In an eighth embodiment of this invention, a process for producing a dilute ethylene stream and dilute propylene stream is provided as in Figure 8.

Step (1) is separating the cracked gas stream in line 800 in a depropanizer zone 805 to produce a C₃- stream in line 810 and a C4+ stream in line 845. The depropanizer zone 805 comprises a fractionator sufficient to produce the C₃- stream in line 810 and the C₄+ stream in line 845. The C₃- stream in line 810 comprises hydrogen, methane, ethane, ethylene, acetylene, propane, propylene, methylacetylene and propadiene. The amount of propylene in the C₃- stream in line 810 is in a range of about 1 % to about 32% by weight, preferably, in a range of 15% to 30% by weight. The C₄+ stream in line 845 comprises C₄ hydrocarbons and heavier constituents.

Step (2) is separating the C₃- stream in line 810 in a deethanizer zone 815 to produce a C₂- stream in line 820 and a C₃ stream in line 890. The deethanizer zone 815 comprises a fractionator sufficient to produce the C₂- stream in line 820 and a C₃ stream in line 890. The C₂- stream comprises hydrogen, methane, ethane, acetylene, and ethylene. The C₃ stream comprises C₃ hydrocarbons.

Step (3) is hydrogenating the C₂- stream in line 820 in a hydrogenation zone 825 to remove , a portion of the acetylene to produce the dilute ethylene stream in line 830. The hydrogenating zone 825 is the same as previously described in the first embodiment.

Generally, the amount of ethylene in the dilute ethylene stream in line 830 is in a range of 5 about 30% to about 60% by weight, preferably, 40% to 60 % by weight. The dilute ethylene stream in line 830 then can be routed to an dilute ethylene derivative unit 835 to produce different chemicals in line 840 including, but not limited to, ethylbenzene. The dilute ethylene derivative unit 835 is the same as dilute ethylene derivative unit 35 previously described in the first embodiment. Optionally, an affluent gas stream in line 836 from the dilute ethylene derivative unit 835 can be recycled to a cracking zone 105 as shown in Figure 2 for the production of more dilute ethylene.

Step (4) is reacting the C₃ stream in line 890 in a MAPD reactor zone 895 to remove a portion of methylacetylene and propadiene to produce the dilute propylene stream in line 900. The MAPD reactor zone 895 and the dilute propylene in line 900 are previously described in the first embodiment.

The dilute propylene stream in line 900 then can be routed to a dilute propylene derivative unit 905 to produce different dilute propylene derivatives in line 910. The dilute propylene derivative unit 905 is the same as dilute propylene derivative unit 70 previously described in the first embodiment.

Optionally, the C₄+ stream in line 845 is separated in a debutanizer zone 850 to produce a C₄ stream in line 855 and a C₅+ stream in line 860. The debutanizer zone 850 comprises a fractionator sufficient to produce the C₄ stream in line 855 and a C₅+ stream in line 860. The debutanizer zone 850 and the C₄ stream in line 855, and then C₅+ stream in line 860 are the same as previously described in the first embodiment.

Optionally, the C₅+ stream in line 860 is treated in a hydrotreating zone 865 to produce a C₅ diolefins stream in line 870, a BTX stream in line 875, a DCPD stream in line 880, and a fuel oil stream in line 885. The hydrotreating zone 865, the C₅ diolefins stream in line 870, the BTX stream in line 875, the DCPD stream in line 880 and the fuel oil stream in line 885 are the same as previously described in the first embodiment.

In another aspect of this invention, the second embodiment, which provides a preferred process of producing the cracked gas stream, can be combined with either the first, third, fourth, and eight embodiments to yield one continuos process for producing the dilute ethylene stream and dilute propylene stream. Also second embodiment, can be combined with either the fifth sixth or seventh embodiment to yield one continuous process for producing the dilute ethylene stream.

In another aspect of this invention, the dilute ethylene stream in the eight embodiment previously described is routed to an ethylbenzene process. Processes to produce, ethylbenzene are disclosed in U.S patent numbers 5,602,290; 5,880,320; 5,856,607; 6,252,126. .

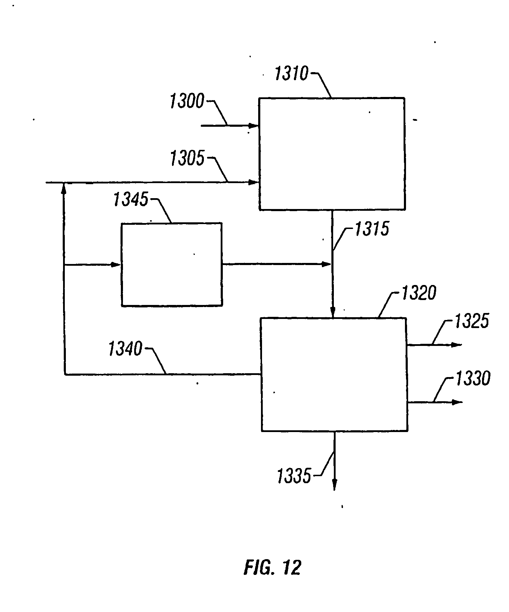

An example of utilizing the dilute ethylene stream to produce ethylbenzene is shown in Figure 12.

Step (1) comprises reacting a dilute ethylene stream in line 1300 with a benzene stream in line 1305 in an alkylation reactor zone 1310 to form an ethylbenzene rich stream, line 1315. The ethylbenzene rich stream in line 1315 comprises benzene and ethylbenzene. The reacting can be accomplished by any means known in the art. For example, the selectivity of converting ethylene to ethylbenzene is greater than 99%. The catalysts used are zeolite catalysts such as a ZSM based zeolite system.

Step (2) comprises separating the ethylbenzene rich stream in line 1315 in a ethylbenzene separation zone 1320 to form a separations tailgas stream in line 1325, an ethylbenzene stream in line 1330, a diethylbenzene and polyethylenebenzene stream in line 1335, and a separation benzene recycle stream in line 1340. Separating can be accomplished by any means know in the art. Generally the separating in the ethylbenzene separation zone comprises at least one fractionator.

Step (3) comprises reacting a portion of the separation benzene recycle stream in line 1340 in a transalkylating reactor zone 1345 to produce an ethylbenzene rich stream in line 1315 The reacting can be accomplished by any means known in the art. For example, the selectivity of converting ethylene to ethylbenzene is greater than 99%. The catalysts use are zeolite based catalysts such as the Washington Group TRA-1 or Lummus Y -zeolite based catalyst system.

Step (4) is recycling a portion of the separation benzene recycle stream in line 1340 to be combined with the benzene stream in line 1305.

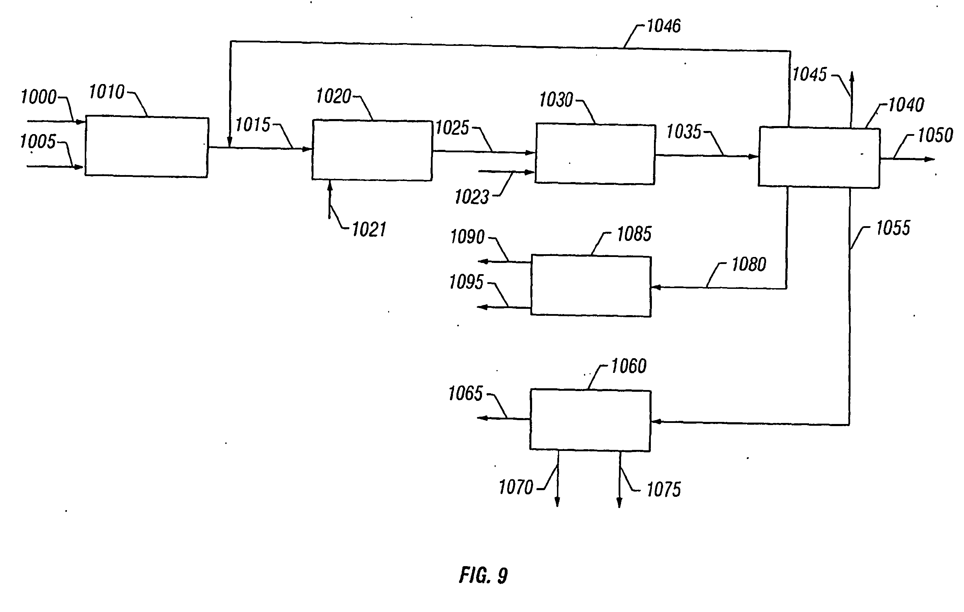

In another aspect of this invention, the dilute propylene stream produced in the embodiments described is routed to a propylene oxide process. Processes to produce propylene oxide product are disclosed in U.S. Patent 3,849,451. An example of this process comprises the following steps is shown in Figure 9.

Step (1) comprises reacting a dilute ethylene stream in line 1000 and a benzene stream in line 1005 in an ethylbenzene reactor zone 1010 to form an ethylbenzene stream in line 1015. The ethylbenzene reactor zone 1010 comprises process equipment sufficient to produce the ethylbenzene stream in line 1015. Typically, the ethylbenzene zone 1010 comprises an alkylation reactor, a transalkylation reactor, and a separation zone to produce ethylbenzene and other products.

Step (2) comprises oxidizing, the ethylbenzene stream in line 1015 with air in line 1021 in an EB oxidation zone 1020 to form an ethylbenzene hydroperoxide (EBHP) stream in line 1025 comprising C₆H₅CH(CH₃)OOH. Oxidation of the ethylbenzene stream in line 1015 can be accomplished by any means know in the art The temperature and pressure in the EB oxidation zone 1020 is that which is sufficient to substantially oxidize the ethylbenzene in line 1015. Preferably, the oxidation occurs at a temperature in the range of about 130 °C to about 160 °C and at an absolute pressure in the range of about 40 psi (0.3MPa) to about 60 psi (60.4MPa)

Step (3) comprises reacting the EBHP stream in line 1025 and a dilute propylene stream in line 1023 in a propylene epoxidation zone to form an impure propylene oxide stream in line 1035 comprising C₃H₆O and methylbenzyl alcohol (MBA), C₆H₅CH(CH₃)OH. The reaction that occurs in the propylene epoxidation zone is:

C₆H₅CH(CH₃)OOH + C₃H₆ → C₃H₆O + C₆H₅CH(CH₃)OH

The temperature and pressure in the propylene epoxidation zone 1030 is that which is sufficient to react the EBHP stream with the dilute propylene stream to form an impure propylene oxide stream. Preferably, the reaction occurs at a temperature in a range of about 60 °C to about 120 °C and at a pressure in a range of about 140 psia to about 700 psia. The reaction in the propylene epoxidation zone can be accomplished by any means know in the art Generally, the reaction of the EBHP stream in line 1025 and the dilute propylene stream in line 1023 is accomplished using a molybdenum catalyst solution.

Step (4) comprises separating the impure propylene oxide stream in line 1035 in a product separator zone 1040 to form a tail gas stream in line 1045, a residue stream in line 1050, a raw propylene oxide stream in line 1080, a MBA/acetophone(ACP) stream in line 1055, and an ethylbenzene recycle stream in line 1046. The product separator zone 1040 comprises equipment sufficient to produce the tail gas stream in line 1045, a residue stream in line 1050, a raw propylene oxide stream in line 1080, and a MBA/ACP stream in line 1055. The ethylbenzene recycle stream in line 1046 is recycled back to be combined with the ethylbenzene stream in line 1015. Generally, the product separation zone comprises at least one fractionator. The MBA/ACP stream in line 1055 comprises C₆H₅CH(CH₃)OH and C₆H₅COCH₃. The tail gas comprises propylene and propane. The residue stream in line 1050 typically comprises benzoic acid, naphthenic acid, and heavier organic compounds.

Step (5) comprises separating the raw propylene oxide stream in a propylene oxide separations zone 1085 to form a propylene oxide stream in line 1095 and an impurities stream in line 1090. The separation of the raw propylene oxide in line 1080 to form an impurities stream in 1090 and a propylene oxide stream in 1095 can be accomplished by any means known in the art.

Step (6) comprises reacting the MBA/ACP stream in line 1055 in a styrene production and separation zone 1060 to form a styrene stream in line 1065, a fuel stream in line 1070, and a wastewater stream in line 1075. The reactions that occur in the styrene production and separation zone 1060 comprise the following:

C₆H₅COCH₃ + H₂ → C₆H₅CHCH₃OH

C₆H₅CHCH₃OH → C₆H₅CH=CH₂ + H₂O

The styrene production and separation zone 1060 comprises equipment sufficient to produce the styrene stream in line 1065, the fuel stream in line 1070, and the wastewater stream in line 1075.

In another aspect of this invention the acrylic acid is produced. Processes to produce acrylic acid are disclosed in U.S Patents 6,281,384 and 6,069,271.

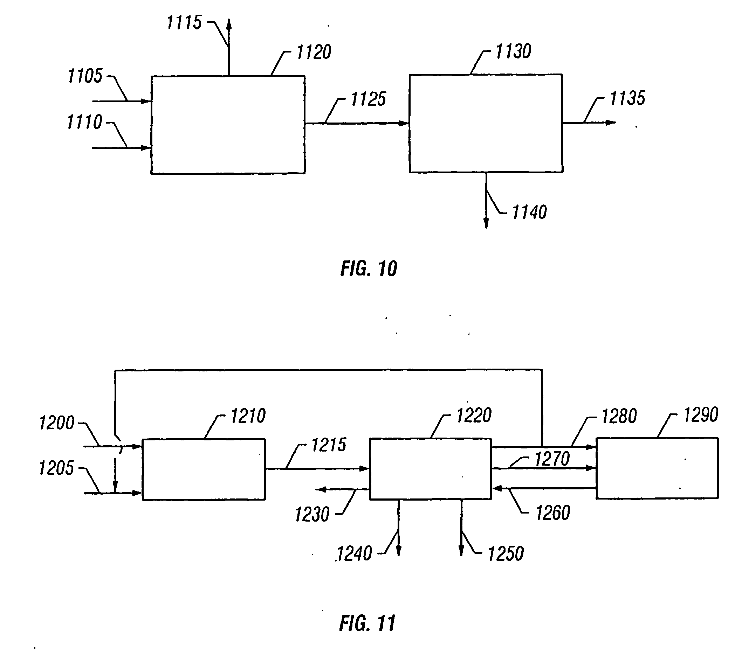

An example of this process comprises the following steps as shown in Figure 10.

Step (1) comprises oxidizing a dilute propylene in line 1105 with air in line 1110 in an oxidation reactor zone 1120 to produce a vent gas stream in line 1115 and an aqueous acrylic acid stream in line 1125. The oxidizing of the dilute propylene comprises the following reactive steps.
(1) C₃H₆ + O₂ → CH₂CHCHO + H₂O
(2) CH₂CHCHO+ ½ O₂ → CH₂CHCOOH

The oxidation reactor zone comprises equipment sufficient to produce the vent gas stream in line 1115 and the aqueous acrylic: acid stream in 1125. For example, the oxidation reactor zone comprises at least one multi-tubular reactor. For example, reaction step (1) can be accomplished through the use of a multi-tubular reactor using catalysts comprising molybdenum and at least one element selected from the group bismuth, tellurium, and tungsten at a temperature of about 300 °C to about 460 °C. The reactive step (2) can be accomplished by a multi-tubular reactor using catalysts comprising molybdenum and vanadium oxide at a temperature of about 240°C to about 450°C.

Step (2) is separating that aqueous acrylic acid stream in line 1125 in a recovery and purification zone 1130 to produce an acrylic acid stream in line 1135 and a mixed acid/ester waste stream in line 1140. The mixed acid/ester waste stream in line 1140 comprises water, formic acid, acetic acid, propionic acid, acrylic acid and ethyl acetate. The recovery and purification zone comprises process equipment sufficient to produce the acrylic acid stream in line 1135 and a mixed acid/ester stream in line 1140. Typically, the recovery and purification zone comprises at least one fractionator.

In another aspect of this invention, the dilute propylene stream is utilized as a feedstock to produce cumene. Processes to produce cumene are disclosed in U.S Patent numbers 5,081,323 and 5,149,894. An example of this process involves the following process steps as shown in Figure 11.

Step (1) comprises reacting a dilute propylene stream in line 1200 with a benzene feed stream in line 1205 in a dilute propylene alkylation zone 1210 to produce a raw cumene stream in line 1215. This step can be accomplished by any means know in the art.

Step (2) is separating the raw cumene steam in line 1215 in a cumene separations zone 1220 to produce a heavies stream in line 1240, the cumene stream in line 1250, a dipropylbenzene stream in line 1270, and a benzene stream in line 1280. Separating can be accomplished by any means know in the art. A portion of the benzene stream in line 1280 can be recycled and combined with the benzene feed stream in line 1205.

Step (3) is reacting the benzene stream in line 1280 and the dipropylbenzene stream in line 1270 in a transalkylation reactor zone 1290 to form a transalkylated cumene-rich stream in line 1260.

Step (4) is separating the transalkylated cumene rich stream in line 1260 in the cumene separations zone 1220 to produce the cumene stream in line 1250, the heavies stream in line 1240, the propane stream in line 1230, the benzene stream in line 1280, and the dipropylbenzene stream in line 1270. A portion of the benzene stream in line 1280 can be recycled and combined in the benzene feed stream in line 1205.

Optionally, the propane stream in line 1230 can be recycled back to the cracking zone 105 shown in Figure 2.

## Claims

1. A process for producing as dilute ethylene stream from a cracked gas stream comprising hydrogen, methane, C₂ hydrocarbon, C₃ hydrocarbon and heavier constituents, said process comprising the following steps in the order named:
(1) separating said cracked gas stream comprising hydrogen, methane, C₂ hydrocarbon, C₃ hydrocarbon and heavier constituent in a deethanizer zone to produce a C₂- stream and a C₃+ stream;
(2) hydrogenating said C₂- stream in a hydrogenation zone to remove a portion of the acetylene to produce said dilute ethylene stream; and
(3) routing said C₃+ stream to storage or other process unit.

2. A process according to claim 1 wherein said C₂- stream is compressed in a C₂- stream-compression zone to form a pressurized C₂- stream which is hydrogenated in step (2).

3. A process for producing a dilute ethylene stream from a cracked gas stream comprising hydrogen, methane, C₂ hydrocarbon, C₃ hydrocarbon and heavier constituent, said process comprising the following steps in the order named:
(1) hydrogenating a portion of the acetylene in said cracked gas stream in a hydrogenation zone to produce a reduced acetylene cracked gas stream;
(2) separating said reduced acetylene cracked gas stream in a deethanizer zone to produce said dilute ethylene stream and a C₃+ stream;
(3) routing said C₃+ stream to storage or other process unit.

4. A process according to claims 1, 2 or 3 which is additionally for producing a dilute propylene stream from the cracked gas stream, wherein in step (3) said C₃+ stream is separated in a depropanizer zone to produce a C₃ stream and a C₄+ stream; and wherein said C₃ stream is reacted in a MAPD reactor zone to convert a portion of methylacetylene and propadiene to propylene and propane to produce said dilute propylene stream.

5. A process for producing a dilute ethylene stream and a dilute propylene stream, said process comprising the following steps in the order named:
(1) separating a cracked gas stream comprising hydrogen, methane, C₂ hydrocarbon, C₃ hydrocarbons and heavier constituents in a depropanizer zone to form a C₃- stream and a C₄+ stream;
(2) separating said C₃- stream in a deethanizer zone to form a C₂- stream and a C₃ stream;
(3) hydrogenating a portion of the acetylene in said C₂- stream in a hydrogenation zone to produce a dilute ethylene stream; and
(4) reacting said C₃ stream in a MAPD zone to convert a portion of methylacetylene and propadiene to propylene and propane to produce said dilute propylene stream.

6. A process according to claim 1, 2, 3, 4 or 5 wherein said cracked gas stream is produced by a process comprising:
(1) heating a hydrocarbon feed in a cracking zone to form a raw cracked gas stream; wherein said raw cracked gas stream comprises hydrogen, methane, C₂ hydrocarbons, C₃ hydrocarbons and heavier constituents;
(2) quenching said raw cracked gas stream in a quenching zone to produce a quenched, cracked gas stream;
(3) compressing said quenched, cracked gas stream in a cracked gas stream-compression zone to form a pressurized, cracked gas stream;
(4) deacidifying said pressurized, cracked gas stream in a deacidifying zone to remove a portion of the hydrogen sulfide to form a wet cracked gas stream; and
(5) drying said wet cracked gas stream in a drying zone to form a cracked gas stream.

7. A process according to claim 4, 5 or 6, further comprising separating said C₄+ stream in a debutanizer zone to produce a C₄ stream and a C₅+ stream.

8. A process according to claim 1, 2, 3, 4, 5 or 6 further comprising passing said dilute ethylene stream to a dilute ethylene derivative unit.

9. A process according to claim 8 wherein said dilute ethylene derivative unit produces ethylbenzene.

10. A process according to claim 4, 5 or 6, further comprising passing said dilute propylene stream to a dilute propylene derivative unit.

11. A process according to claim 10 wherein said dilute propylene derivative unit produces cumene, acrylic acid or propylene oxide.

12. A process according to claim 7 further comprising treating said C₅+ stream in a hydrotreating zone to produce a C₅ diolefins stream, a BTX stream, a DCPD stream and a fuel oil stream.

13. A process according to claim 6 wherein said hydrocarbon feed is selected from the group consisting of ethane, propane, butanes, pentanes, naphtha, and mixtures thereof.

14. A process according to claim 6 wherein said hydrocarbon feed consists essentially of C₅ hydrocarbons.

15. A process according to any preceding claim wherein a propylene oxide stream is produced by a process comprising the following steps:
(1) reacting said dilute ethylene with benzene in an ethylbenzene reactor zone to form an ethylbenzene stream;
(2) oxidizing said ethylbenzene stream with air in an EB oxidation zone to form a EBHP stream;
(3) reacting said EBHP stream with a dilute propylene stream in a propylene epoxidation zone to form an impure propylene oxide stream;
(4) separating said impure propylene oxide stream in a product separator zone to form a raw propylene oxide stream, a MBN/ACP stream, a tail gas stream, and a residue stream; and
(5) separating said raw propylene oxide stream in a propylene oxide separations zone to form an impurities stream and said propylene oxide stream; and
(6) reacting said MBN/ACP stream in a styrene production and separation zone to form a styrene stream, a fuel stream, and a wastewater stream.

16. A process according to claim 4 or 5, or any one of claims 6 to 14 when dependent on claim 4 or 5, wherein an acrylic acid stream is produced by a process comprising the following steps:
(1) oxidizing said dilute propylene stream in a oxidation reactor zone to form a aqueous acrylic acid stream and a vent gas stream; and
(2) seperating said aqueous acrylic acid stream in a recovery and purification zone to form said acrylic acid stream and a mixed acid/ester waste stream.

17. A process according to claim 4 or 5, or any one of claims 6 to 14 when dependent on claim 4 or 5 wherein a cumene stream is produced by a process comprising the following steps:
(1) reacting a dilute propylene stream and a benzene feed stream in a dilute propylene alkylation zone to produce a raw cumene stream;
(2) separating said raw cumene stream in a cumene separations zone to form a benzene stream, a heavies stream, said cumene: stream, a dipropyl benzene stream, and a propane stream.
(3) transalkylating said benzene stream and dipropyl benzene stream in a transalkylation zone to form a transalkylated cumene rich stream;
(4) separating said transalkylated cumene-rich stream in said cumene separations zone to produce said cumene stream, said propane stream, said heavies stream and said benzene stream; and
(5) optionally, recycling a portion of said benzene stream to said dilute propylene alkylation zone

18. A process according to claim 4 or 5, or any one of claims 6 to 14 when dependent on claim 4 or 5, wherein a ethylbenzene stream is produced by a process comprising the following steps;
(1) reacting a dilute ethylene stream and a benzene stream in an alkylation reactor zone to form an ethylbenzene rich stream
(2) separating said ethylbenzene rich stream in a ethylbenzene separation zone to form a separations benzene recycle stream, a separations tail gas stream, a diethylbenzene and polyethylbenzene stream, and a ethylbenzene stream;
(3) reacting said separations benzene recycle stream in an ethylbenzene transalkylation reactor zone to produce said ethylbenzene rich stream; and
(4) optionally, recycling a portion of said separations benzene recycle stream to said dilute propylene alkylation reactor zone.

## Patentansprüche

1. Ein Verfahren zur Herstellung eines verdünnten Ethylenstroms aus einem gecrackten Gasstrom, der Wasserstoff, Methan, C₂-Kohlenwasserstoffe, C₃-Kohlenwasserstoffe und schwerere Bestandteile umfasst, wobei das Verfahren die folgenden Schritte in der genannten Reihenfolge umfasst:
(1) Trennen des gecrackten Gasstroms, der Wasserstoff, Methan, C₂-Kohlenwasserstoffe, C₃-Kohlenwasserstoffe und schwerere Bestandteile umfasst, in einer Deethanisierungszone, um einen C₂₋-Strom und einen C₃₊-Strom zu erzeugen;
(2) Hydrieren des C₂₋-Stroms in einer Hydrierungszone, um einen Teil des Acetylens zur Erzeugung des verdünnten Ethylenstroms zu entfernen; und
(3) Leiten des C₃₊-Stroms zu einer Speicher- oder anderen Prozesseinheit.

2. Ein Verfahren nach Anspruch 1, wobei der C₂₋-Strom in einer Verdichtungszone des C₂₋-Stroms zur Bildung eines unter Druck stehenden C₂₋-Stroms verdichtet wird, der in Schritt (2) hydriert wird.

3. Ein Verfahren zur Herstellung eines verdünnten Ethylenstroms aus einem gecrackten Gasstrom, der Wasserstoff, Methan, C₂-Kohlenwasserstoffe, C₃-Kohlenwasserstoffe und schwerere Bestandteile umfasst, wobei das Verarbeiten die folgenden Schritte in der genannten Reihenfolge umfasst:
(1) Hydrieren eines Teils des Acetylens in dem gecrackten Gasstrom in einer Hydrierungszone zur Erzeugung eines gecrackten Gasstroms mit reduziertem Acetylen;
(2) Trennen des gecrackten Gasstroms mit reduziertem Acetylen in einer Deethanisierungszone zur Erzeugung des verdünnten Ethylenstroms und eines C₃₊-Stroms;
(3) Leiten des C₃₊-Stroms zu einer Speicher- oder anderen Prozesseinheit.

4. Ein Verfahren nach Anspruch 1, 2 oder 3, das zusätzlich zur Herstellung eines verdünnten Propylenstroms aus dem gecrackten Gasstrom ist, wobei in Schritt (3) der C₃₊-Strom in einer Depropanisierungszone zur Erzeugung eines C₃-Stroms und eines C₄₊-Stroms abgetrennt wird; und wobei der C₃-Strom in einer MAPD-Reaktorzone umgesetzt wird, um einen Teil des Methylacetylens und Propadiens in Propylen und Propan zur Erzeugung des verdünnten Propylenstroms umzuwandeln.

5. Ein Verfahren zur Herstellung eines verdünnten Ethylenstroms und eines verdünnten Propylenstroms, wobei das Verfahren die folgenden Schritte in der genannten Reihenfolge umfasst:
(1) Trennen eines gecrackten Gasstroms, der Wasserstoff, Methan, C₂-Kohlenwasserstoffe, C₃-Kohlenwasserstoffe und schwerere Bestandteile umfasst, in einer Depropanisierungszone zur Bildung eines C₃₋-Stroms und eines C₄₊-Stroms;
(2) Trennen des C₃₋-Stroms in einer Deethanisierungszone zur Bildung eines C₂₋-Stroms und eines C₃-Stroms;
(3) Hydrieren eines Teils des Acetylens in dem C₂₋-Strom in einer Hydrierungszone zur Erzeugung eines verdünnten Ethylenstroms; und
(9) Umsetzen des C₃-Stroms in einer MAPD-Zone, um einen Teil des Methylacetylens und Propadiens in Propylen und Propan zur Erzeugung des verdünnten Propylenstroms umzuwandeln.

6. Ein Verfahren nach Anspruch 1, 2, 3, 4 oder 5, wobei der gecrackte Gasstrom durch ein Verfahren erzeugt wird, das folgendes umfasst:
(1) Erwärmen einer Kohlenwasserstoffeinspeisung in einer Crackzone zur Bildung eines rohen gecrackten Gasstroms; wobei der rohe gecrackte Gasstrom Wasserstoff, Methan, C₂-Kohlenwasserstoffe, C₃-Kohlenwasserstoffe und schwerere Bestandteile umfasst;
(2) Quenchen des rohen gecrackten Gasstroms in einer Quenchzone zur Erzeugung eines gequenchten, gecrackten Gasstroms;
(3) Verdichten des gequenchten, gecrackten Gasstroms in einer Verdichtungszone des gecrackten Gasstroms zur Bildung eines unter Druck stehenden, gecrackten Gasstroms;
(4) Entsäuern des unter Druck stehenden, gecrackten Gasstroms in einer Entsäuerungszone, um einen Teil des Schwefelwasserstoffs zur Bildung eines nassen gecrackten Gasstroms zu entfernen; und
(5) Trocknen des nassen gecrackten Gasstroms in einer Trocknungszone zur Bildung eines gecrackten Gasstroms.

7. Ein Verfahren nach Anspruch 4, 5 oder 6, das ferner das Trennen des C₄₊-Stroms in einer Debutanisierungszone zur Erzeugung eines C₄-Stroms und eines C₅₊-Stroms umfasst.

8. Ein Verfahren nach Anspruch 1, 2, 3, 4, 5 oder 6, das ferner das Weiterleiten des verdünnten Ethylenstroms zu einer Einheit eines verdünnten Ethylenderivats umfasst.

9. Ein Verfahren nach Anspruch 8, wobei die Einheit des verdünnten Ethylenderivats Ethylbenzol erzeugt.

10. Ein Verfahren nach Anspruch 4, 5 oder 6, das ferner das Weiterleiten des verdünnten Propylenstroms zu einer Einheit eines verdünnten Propylenderivats umfasst.

11. Ein Verfahren nach Anspruch 10, wobei die Einheit des verdünnten Propylenderivats Cumen, Acrylsäure oder Propylenoxid erzeugt.

12. Ein Verfahren nach Anspruch 7, das ferner das Behandeln des C₅₊-Stroms in einer Hydrierbehandlungszone zur Erzeugung eines C₅-Diolefinstroms, eines BTX-Stroms, eines DCPD-Stroms und eines Heizölstroms umfasst.

13. Ein Verfahren nach Anspruch 6, wobei die KohlenwasserstoffEinspeisung aus der Gruppe ausgewählt ist, die aus Ethan, Propan, Butanen, Pentanen, Naphtha und Gemischen davon besteht.

14. Ein Verfahren nach Anspruch 6, wobei die Kohlenwasserstoffeinspeisung im Wesentlichen aus C₅-Kohlenwasserstoffen besteht.

15. Ein Verfahren nach einem vorstehenden Anspruch, wobei ein Propylenoxidstrom durch ein Verfahren erzeugt wird, das die folgenden Schritte umfasst:
(1) Umsetzen des verdünnten Ethylens mit Benzol in einer Ethylbenzol-Reaktorzone zur Bildung eines Ethylbenzolstroms;
(2) Oxidieren des gleichen Ethylbenzolstroms mit Luft in einer EP-Oxidationszone zur Bildung eines EBHP-Stroms;
(3) Umsetzen des EBHP-Stroms mit einem verdünnten Propylenstrom in einer Propylenepoxidierungszone zur Bildung eines nicht reinen Propylenoxidstroms;
(4) Trennen des nicht reinen Propylenoxidstroms in einer Produktabtrennungszone zur Bildung eines rohen Propylenoxidstroms, eines MBN/ACP-Stroms, eines Endgasstroms und eines Reststroms; und
(5) Trennen des rohen Propylenoxidstroms in einer Propylenoxid-Abtrennungszone zur Bildung eines Verunreinigungsstroms und des Propylenoxidstroms; und
(6) Umsetzen des MBN/ACP-Stroms in einer Styrol-Herstellungsund Abtrennungszone zur Bildung eines Styrolstroms, eines Brennstoffstroms und eines Schmutzwasserstroms.

16. Ein Verfahren nach Anspruch 4 oder 5, oder nach einem der Ansprüche 6 bis 14 bei Abhängigkeit von Anspruch 4 oder 5, wobei ein Acrylsäurestrom durch ein Verfahren erzeugt wird, das die folgenden Schritte umfasst:
(1) Oxidieren des verdünnten Propylenstroms in einer Oxidationsreaktorzone zur Bildung eines wässrigen Acrylsäurestroms und eines Abgasstroms; und
(2) Trennen des wässrigen Acrylsäurestroms in einer Rückgewinnungs- und Reinigungszone zur Bildung des Acrylsäurestroms und eines gemischten Säure/Ester-Abfallstroms.

17. Ein Verfahren nach Anspruch 4 oder 5, oder nach einem der Ansprüche 6 bis 14 bei Abhängigkeit von Anspruch 4 oder 5, wobei ein Cumenstrom durch ein Verfahren erzeugt wird, das die folgenden Schritte umfasst:
(1) Umsetzen eines verdünnten Propylenstroms und eines Benzol-Einspeisungsstroms in einer verdünnten Propylen-Alkylierungszone zur Erzeugung eines rohen Cumenstroms;
(2) Trennen des rohen Cumenstroms in einer Cumen-Abtrennungszone zur Bildung eines Benzolstroms, eines Stroms hochsiedender Komponenten, des Cumenstroms, eines Dipropylbenzolstroms und eines Propanstroms;
(3) Transalkylieren des Benzolstroms und Dipropylbenzolstroms in einer Transalkylierungszone zur Bildung eines an transalkyliertem Cumen reichen Stroms;
(4) Trennen des an transalkyliertem Cumen reichen Stroms in der Cumen-Abtrennungszone zur Erzeugung des Cumenstroms, des Propanstroms, des Stroms hochsiedender Komponenten und des Benzolstroms; und
(5) wahlweise, Rückführen eines Teils des Benzolstroms in die verdünnte Propylen-Alkylierungszone.

18. Ein Verfahren nach Anspruch 4 oder 5, oder nach einem der Ansprüche 6 bis 14 bei Abhängigkeit von Anspruch 4 oder 5, wobei ein Ethylbenzolstrom durch ein Verfahren erzeugt wird, das die folgenden Schritte umfasst:
(1) Umsetzen eines verdünnten Ethylenstroms und eines Benzolstroms in einer Alkylierungsreaktorzone zur Bildung eines an Ethylbenzol reichen Stroms;
(2) Trennen des an Ethylbenzol reichen Stroms in einer Ethylbenzol-Abtrennungszone zur Bildung eines abgetrennten Benzol-Rückführungsstroms, eines abgetrennten Endgasstroms, eines Diethylbenzolund Polyethylbenzolstroms und eines Ethylbenzolstroms;
(3) Umsetzen des abgetrennten Benzol-Rückführungsstroms in einer Ethylbenzol-Transalkylierungsreaktorzone zur Erzeugung des an Ethylbenzol reichen Stroms; und
(4) wahlweise, Rückführen eines Teils des abgetrennten Benzol-Rückführungsstroms in die verdünnte Propylen-Alkylierungsreaktorzone.

## Revendications

1. - Procédé de production d'un courant d'éthylène dilué à partir d'un courant de gaz craqué comprenant de l'hydrogène, du méthane, des hydrocarbures en C₂, des hydrocarbures en C₃ et des constituants plus lourds, ledit procédé comprenant les étapes suivantes dans l'ordre nommé :
(1) séparer ledit courant de gaz craqué comprenant de l'hydrogène, du méthane, des hydrocarbures en C₂, des hydrocarbures en C₃ et des constituants plus lourds dans une zone de déséthaniseur pour produire un courant de C₂- et un courant de C₃+ ;
(2) hydrogéner ledit courant de C₂- dans une zone d'hydrogénation pour retirer une partie de l'acétylène pour produire ledit courant d'éthylène dilué ; et
(3) acheminer ledit courant de C₃+ dans une unité de stockage ou autre unité de traitement.

2. - Procédé selon la revendication 1, dans lequel ledit courant de C₂- est comprimé dans une zone de compression de courant de C₂- pour former un courant de C₂- pressurisé qui est hydrogéné à l'étape (2).

3. - Procédé de fabrication d'un courant d'éthylène dilué à partir d'un courant de gaz craqué comprenant de l'hydrogène, du méthane, des hydrocarbures en C₂, des hydrocarbures en C₃ et des constituants plus lourds, ledit procédé comprenant les étapes suivantes dans l'ordre nommé :
(1) hydrogéner une partie de l'acétylène dans ledit courant de gaz craqué dans une zone d'hydrogénation pour produire un courant de gaz craqué à acétylène réduit ;
(2) séparer ledit courant de gaz craqué à acétylène réduit dans une zone de déséthaniseur pour produire ledit courant d'éthylène dilué et un courant de C₃+ ;
(3) acheminer ledit courant de C₃+ dans une unité de stockage ou autre unité de traitement.

4. - Procédé selon l'une des revendications 1, 2 ou 3, qui est additionnellement destiné à produire un courant de propylène dilué à partir du courant de gaz craqué, dans lequel, dans l'étape (3), ledit courant de C₃+ est séparé dans une zone de dépropaniseur pour produire un courant de C₃ et un courant de C₄+ ; et dans lequel ledit courant de C₃ est mis à réagir dans une zone de réacteur de MAPD pour convertir une partie de méthylacétylène et de propadiène en propylène et propane pour produire ledit courant de propylène dilué.

5. - Procédé de production d'un courant d'éthylène dilué et d'un courant de propylène dilué, ledit procédé comprenant les étapes suivantes dans l'ordre nommé :
(1) séparer un courant de gaz craqué comprenant de l'hydrogène, du méthane, des hydrocarbures en C₂, des hydrocarbures en C₃ et des constituants plus lourds dans une zone de dépropaniseur pour former un courant de C₃- et un courant de C₄+ ;
(2) séparer ledit courant de C₃- dans une zone de déséthaniseur pour former un courant de C₂- et un courant de C₃ ;
(3) hydrogéner une partie de l'acétylène dans ledit courant de C₂- dans une zone d'hydrogénation pour produire un courant d'éthylène dilué ; et
(4) faire réagir ledit courant de C₃ dans une zone de MAPD pour convertir une partie de méthylacétylène et de propadiène en propylène et propane pour produire ledit courant de propylène dilué.

6. - Procédé selon l'une des revendications 1, 2, 3, 4 ou 5, dans lequel ledit courant de gaz craqué est obtenu par un procédé comprenant :
(1) chauffer une alimentation hydrocarbonée dans une zone de craquage pour former un courant de gaz craqué brut, ledit courant de gaz craqué brut comprenant de l'hydrogène, du méthane, des hydrocarbures en C₂, des hydrocarbures en C₃ et des constituants plus lourds ;
(2) refroidir brusquement ledit courant de gaz craqué brut dans une zone de refroidissement brusque pour produire un courant de gaz craqué, refroidi brusquement ;
(3) comprimer ledit courant de gaz craqué, refroidi brusquement, dans une zone de compression du courant de gaz craqué pour former un courant de gaz craqué, pressurisé ;
(4) désacidifier ledit courant de gaz craqué, pressurisé, dans une zone de désacidification pour éliminer une partie du sulfure d'hydrogène pour former un courant de gaz craqué humide ; et
(5) sécher ledit courant de gaz craqué humide dans une zone de séchage pour former un courant de gaz craqué.

7. - Procédé selon l'une des revendications 4, 5 ou 6, comprenant en outre la séparation dudit courant de C₄+ dans une zone de débutaniseur pour produire un courant de C₄ et un courant de C₅+.

8. - Procédé selon l'une des revendications 1, 2, 3, 4, 5 ou 6, comprenant en outre l'opération consistant à faire passer ledit courant d'éthylène dilué dans une unité de dérivé d'éthylène dilué.

9. - Procédé selon la revendication 8, dans lequel ladite unité de dérivé d'éthylène dilué produit de l'éthylbenzène.

10. - Procédé selon l'une des revendications 4, 5 ou 6, comprenant en outre l'opération consistant à faire passer ledit courant de propylène dilué dans une unité de dérivé de propylène dilué.

11. - Procédé selon la revendication 10, dans lequel ladite unité de dérivé de propylène dilué produit du cumène, de l'acide acrylique ou de l'oxyde de propylène.

12. - Procédé selon la revendication 7, comprenant en outre le traitement dudit courant de C₅+ dans une zone d'hydrotraitement pour produire un courant de dioléfines en C₅, un courant de BTX, un courant de DCPD et un courant de fuel-oil.

13. - Procédé selon la revendication 6, dans lequel ladite alimentation hydrocarbonée est choisie dans le groupe constitué par l'éthane, le propane, les butanes, les pentanes, le naphta et leurs mélanges.

14. - Procédé selon la revendication 6, dans lequel ladite alimentation hydrocarbonée consiste essentiellement en hydrocarbures en C₅.

15. - Procédé selon l'une quelconque des revendications précédentes, dans lequel un courant d'oxyde de propylène est obtenu par un procédé comprenant les étapes suivantes :
(1) faire réagir ledit éthylène dilué avec du benzène dans une zone de réacteur d'éthylbenzène pour former un courant d'éthylbenzène ;
(2) oxyder ledit courant d'éthylbenzène avec de l'air dans une zone d'oxydation d'EB pour former un courant d'EBHP ;
(3) faire réagir ledit courant d'EBHP avec un courant de propylène dilué dans une zone d'époxydation du propylène pour former un courant d'oxyde de propylène impur ;
(4) séparer ledit courant d'oxyde de propylène impur dans une zone de séparateur de produit pour former un courant d'oxyde de propylène brut, un courant de MBN/ACP, un courant de gaz de queue et un courant de résidu ; et
(5) séparer ledit courant d'oxyde de propylène brut dans une zone de séparation d'oxyde de propylène pour former un courant d'impuretés et ledit courant d'oxyde de propylène ; et
(6) faire réagir ledit courant de MBN/ACP dans une zone de production et de séparation de styrène pour former un courant de styrène, un courant de fuel et un courant d'eau résiduaire.

16. - Procédé selon l'une des revendications 4 ou 5, ou selon l'une quelconque des revendications 6 à 14 lorsqu'elle est dépendante de l'une des revendications 4 ou 5, dans lequel un courant d'acide acrylique est obtenu par un procédé comprenant les étapes suivantes :
(1) oxyder ledit courant de propylène dilué dans une zone de réacteur d'oxydation pour former un courant d'acide acrylique aqueux et un courant de gaz évacués ; et
(2) séparer ledit courant d'acide acrylique aqueux dans une zone de récupération et de purification pour former ledit courant d'acide acrylique et un courant résiduaire d'acide/ester mixte.

17. - Procédé selon l'une des revendications 4 ou 5, ou selon l'une quelconque des revendications 6 à 14 lorsqu'elle est dépendante de l'une des revendications 4 ou 5, dans lequel un courant de cumène est obtenu par un procédé comprenant les étapes suivantes :
(1) faire réagir un courant de propylène dilué et un courant d'alimentation de benzène dans une zone d'alkylation de propylène dilué pour produire un courant de cumène brut ;
(2) séparer ledit courant de cumène brut dans une zone de séparation de cumène pour former un courant de benzène, un courant de lourds, ledit courant de cumène, un courant de dipropyl benzène et un courant de propane ;
(3) transalkyler ledit courant de benzène et ledit courant de dipropyl benzène dans une zone de transalkylation pour former un courant riche en cumène transalkylé ;
(4) séparer ledit courant riche en cumène transalkylé dans ladite zone de séparation de cumène pour produire ledit courant de cumène, ledit courant de propane, ledit courant de lourds et ledit courant de benzène ; et
(5) facultativement, recycler une partie dudit courant de benzène dans ladite zone d'alkylation de propylène dilué.

18. - Procédé selon l'une des revendications 4 ou 5, ou selon l'une quelconque des revendications 6 à 14 lorsqu'elle est dépendante de l'une des revendications 4 ou 5, dans lequel un courant d'éthylbenzène est obtenu par un procédé comprenant un procédé comprenant les étapes suivantes :
(1) faire réagir un courant d'éthylène dilué et un courant de benzène dans une zone de réacteur d'alkylation pour former un courant riche en éthylbenzène ;
(2) séparer ledit courant riche en éthylbenzène dans une zone de séparation d'éthylbenzène pour former un courant de recyclage de benzène de séparation, un courant de gaz de queue de séparation, un courant de diéthylbenzène et de polyéthylbenzène et un courant d'éthylbenzène ;
(3) faire réagir ledit courant de recyclage de benzène de séparation dans une zone de réacteur de transalkylation d'éthylbenzène pour produire ledit courant riche en éthylbenzène ; et
(4) facultativement, recycler une partie dudit courant de recyclage de benzène de séparation dans ladite zone de réacteur d'alkylation de propylène dilué.
